# DEMANDE DE BREVET EUROPEEN

(11) **EP 1 891 932 A2**
(43) Date de publication de la demande: **27.02.2008**
(21) Numéro de dépôt: 07301197.5
(22) Date de dépôt: 02.07.2007
(51) Int. Cl.: A61K 8/60, A61K 8/81, A61Q 19/08

(54) **Composition associant un dérivé C-glycoside et un polymère émulsionnant**

(30) Priorité: 03.07.2006 FR 0606023
(71) Demandeur: L'ORÉAL, 75008 Paris (FR)
(72) Inventeur: Simonnet, Jean-Thierry, 94230, CACHAN (FR); Chevalier, Véronique, 94440, VILLECRESNES (FR)
(74) Mandataire: Le Coupanec, Pascale A.M.P.

(57) **Abrégé**

La présente invention concerne une composition cosmétique et/ou dermatologique comprenant, dans un milieu physiologiquement acceptable, au moins un dérivé C-glycoside et au moins un polymère émulsionnant choisi parmi les polyoléfines à partie(s) polaire(s) et les polymères amphiphiles comportant au moins un motif acide acrylamido-2-méthyl propane sulfonique (AMPS).

## Description

La présente invention concerne des compositions cosmétiques et/ou dermatologiques comprenant au moins un dérivé C-glycoside et au moins un polymère émulsionnant. Les compositions selon l'invention sont notamment destinées à améliorer l'efficacité biologique dudit dérivé.

Les compositions selon l'invention sont en particulier destinées au soin et/ou au maquillage des matières kératiniques et notamment de la peau.

Les dérivés C-glycosides sont connus pour présenter des activités biologiques intéressantes au niveau cutané. Des dérivés C-glycosides ont ainsi déjà été décrits dans le document WO 02/051828 comme ayant la propriété de stimuler la synthèse des glycosaminoglycannes, contribuant ainsi à maintenir entre autre une bonne hydratation et une bonne souplesse de la peau.

La peau est connue pour constituer une barrière naturelle remarquablement efficace pour éviter la pénétration de corps et molécules étrangères à l'organisme mais aussi la déshydratation de celui-ci, par exemple. En conséquence, pour qu'un produit cosmétique contenant un dérivé C-glycoside, dont la cible se situe dans l'épiderme vivant, soit efficace, il est nécessaire que ce dérivé puisse y être véhiculé en quantité suffisante tout en garantissant une parfaite innocuité de la préparation au niveau cutané, mais également un agrément cosmétique incontestable.

Des propénétrants tel que le Transcutol^{®} ou l'éthanol à forte concentration sont déjà utilisés, notamment dans le domaine pharmaceutique. Toutefois, bien qu'efficaces en terme d'effet véhicule, ces deux composés ne donnent pas totalement satisfaction pour ce qui est des autres propriétés mentionnées ci-dessus.

Il demeure donc un besoin d'une composition cosmétique, notamment dermatologique, comprenant un dérivé C-glycoside en une quantité suffisante, et qui soit dénuée des inconvénients précités. La présente invention a précisément pour objet une telle composition.

Les inventeurs ont en effet découvert, de façon inattendue, que l'association d'un dérivé C-glucoside avec au moins un polymère émulsionnant choisi parmi les polyoléfines à partie(s) polaire(s) et les polymères amphiphiles comportant au moins un motif acide acrylamido-2-méthyl propane sulfonique (AMPS) permettait de résoudre ce problème.

L'invention concerne ainsi, selon un de ses premiers aspects, une composition cosmétique et/ou dermatologique comprenant, dans un milieu physiologiquement acceptable, au moins un dérivé C-glycoside et au moins un polymère émulsionnant choisi parmi les polyoléfines à partie(s) polaire(s) et les polymères amphiphiles comportant au moins un motif acide acrylamido-2-méthyl propane sulfonique (AMPS).

En particulier, une telle composition est avantageusement mise en oeuvre pour le soin et/ou le maquillage des matières kératiniques, et notamment de la peau.

L'association d'un dérivé C-glycoside avec un polymère émulsionnant selon l'invention permet d'obtenir des compositions cosmétiques ou dermatologiques remarquablement efficaces pour prévenir et/ou lutter contre les signes du vieillissement cutané.

Par « signes du vieillissement cutané », on entend toutes les modifications de l'aspect extérieur de la peau dues à un vieillissement chronologique ou photo-induit comme par exemple les rides, la peau flétrie, la peau molle, la peau amincie, le manque d'élasticité ou de tonus de la peau.

Ainsi, selon un autre de ses aspects, l'invention concerne l'utilisation d'une association d'au moins un dérivé C-glycoside et d'au moins un polymère émulsionnant choisi parmi les polyoléfines à partie(s) polaire(s) et les polymères amphiphiles comportant au moins un motif acide acrylamido-2-méthyl propane sulfonique (AMPS) pour la préparation d'une composition cosmétique et/ou dermatologique destinée à prévenir et/ou lutter contre les signes du vieillissement cutané.

Elle concerne en particulier l'utilisation d'une association d'au moins un dérivé C-glycoside et d'au moins un polymère émulsionnant choisi parmi les polyoléfines à partie(s) polaire(s) et les polymères amphiphiles comportant au moins un motif acide acrylamido-2-méthyl propane sulfonique (AMPS) à titre d'actif cosmétique destiné à prévenir et/ou lutter contre les signes du vieillissement cutané.

Selon encore un autre de ses objets, la présente invention concerne un procédé de traitement non thérapeutique de maquillage et/ou de soin des matières kératiniques, notamment de la peau, comprenant au moins l'étape d'appliquer sur lesdites matières kératiniques, et notamment sur la peau, au moins une couche d'une composition conforme à l'invention.

### POLYMERES EMULSIONNANTS

Par « polymère émulsionnant », on entend désigner, au sens de l'invention, un polymère possédant des propriétés amphiphiles, c'est-à-dire doté d'au moins une partie hydrophile et d'au moins une partie hydrophobe. Les groupements hydrophiles et les groupements hydrophobes sont bien connus de l'homme du métier.

Au sens de la présente invention on entend désigner par "polymère" un composé comprenant au moins deux motifs de répétition, en particulier, au moins cinq motifs de répétition.

Dans le cadre de la présente invention, "matières kératiniques" comprend par exemple la peau et les lèvres.

Un polymère émulsionnant peut notamment être une polyoléfine à partie(s) polaire(s) et/ou au moins un polymère amphiphile comportant au moins un motif acide acrylamido 2-méthylpropane sulfonique (AMPS).

### Polyoléfines à partie(s) polaire(s)

Les polyoléfines à partie(s) polaire(s) utilisables dans la présente invention sont déjà connues dans d'autres domaines et sont par exemple décrites dans les documents US-A-5,129,972 et US-A-4,919,179, comme stabilisants d'émulsions explosives et dans les documents US-A-5,518,517 et US-A-5,858,055 comme stabilisants de compositions fertilisantes.

Les polyoléfines à partie(s) polaire(s) utilisables dans les compositions de l'invention peuvent comporter une partie apolaire polyoléfinique et au moins une partie polaire.

Elles peuvent présenter une structure de type bloc ou peigne.

La partie apolaire polyoléfinique comprend au moins 40 atomes de carbone et en particulier de 60 à 700 atomes de carbone.

Cette partie apolaire polyoléfine peut être choisie parmi les oligomères, les polymères et/ou les copolymères de monomères en C₂ à C₂₀ et notamment d'éthylène, de propylène, de 1-butène, d'isobutène, de 1-pentène, de 2-méthyl-1-butène, de 3-méthyl-1-butène, de 1-héxène, de 1-heptène, de 1-octène, de 1-décène, de 1-undécène, de 1-dodécène, de 1-tridécène, de 1-tetradécène, de 1-pentadécène, de 1-hexadécène, de 1-heptadécène et de 1-octadécène. Ces polyoléfines peuvent être hydrogénées ou non.

Par ailleurs, les polyoléfines à partie(s) polaire(s) utilisables dans les compositions de l'invention, par exemple sous forme d'émulsions, comportent au moins une partie polaire. Cette partie polaire est présente avantageusement pour leur conférer des propriétés amphiphiles.

Ainsi, ces polyoléfines à partie(s) polaire(s) peuvent abaisser la tension interfaciale (eau/huile) d'au moins 10 mN/m lorsqu'elles sont présentes à une concentration de 0,01% en poids par rapport au poids total de la phase huileuse. Par exemple, la polyoléfine à terminaison succinique commercialisée sous la dénomination Lubrizol 2724^{®} par la société Lubrizol, à une concentration de 0,01% en poids par rapport au poids total de la phase huileuse, abaisse la tension interfaciale de 15 mN/m à l'interface d'une phase aqueuse constituée d'une solution aqueuse à 1 % de MgSO₄, et d'une phase huileuse comportant un mélange d'huiles (isohexadécane/polyisobutène hydrogéné/silicone volatile dans un rapport 8/6/4), le rapport de la phase aqueuse et de la phase huileuse étant égal à 1.

La partie polaire des polyoléfines à partie(s) polaire(s) de l'invention peut être anionique, cationique, non ionique, zwitterionique ou amphotère. Elle peut être par exemple constituée de polyalkylène-glycols, de polyalkylène-imines, d'acides ou de diacides carboxyliques, de leurs anhydrides ou de leurs dérivés tels que leurs esters, leurs amides et leurs sels.

Les polyoléfines à partie(s) polaire(s) acide carboxylique peuvent être par exemple, issues de la réaction entre une polyoléfine et au moins un acide ou anhydride carboxylique, éventuellement totalement ou partiellement salifié, choisi dans le groupe comprenant l'acide ou l'anhydride succinique, l'acide maléique, l'anhydride maléique, l'acide fumarique, l'acide itaconique, l'acide citraconique (ou acide méthyl maléique), l'acide mésaconique (ou acide méthyl fumarique), l'acide aconitique, leurs dérivés esters ou amides, et leurs mélanges.

Selon un mode de réalisation, la partie polaire de la polyoléfine peut être choisie dans le groupe comprenant le polyoxyéthylène, l'acide ou l'anhydride succinique, les esters ou amides de l'acide ou de l'anhydride succinique, les sels de métal alcalin ou alcalino-terreux ou sels organiques de l'acide ou de l'anhydride succinique, ou les sels partiels des monoesters ou monoamides de l'acide ou de l'anhydride succinique.

Les polyoléfines à partie(s) polaire(s) polyoxyéthylène(s) peuvent être par exemple choisies parmi les polymères diblocs polyisoprène-polyoxyéthylène, les polymères poly(éthylène-co-propylène)-polyoxyéthylène et leurs mélanges. Ces polymères sont notamment décrits dans la publication de Allgaier et al, Macromolecules, 1997, vol. 30, p.1582-1586.

Les polyoléfines à partie(s) polaire(s) acide ou anhydride succinique peuvent être choisies notamment parmi les dérivés polyoléfines d'acide ou d'anhydride succinique décrits dans les brevets US-A-4,234,435, US-A-4,708,753, US-A-5,129,972, USA-4,931,110, GB-A-2,156,799, US-A-4,877,756 et US-A-4,919,179.

La partie polyoléfine peut être constituée par exemple de polyisobutylène, hydrogéné ou non, de poids moléculaire allant de 400 à 5000 g/mol. Dans le polyisobutylène à terminaison succinique ainsi obtenu, la partie succinique peut être éventuellement modifiée, c'est-à-dire estérifiée, amidifiée ou sous forme de sel. Elle peut être modifiée par des alcools, des amines, des alcanolamines ou des polyols, ou encore se trouver sous forme de sels de métal alcalin ou alcalino-terreux, d'ammonium ou encore de base organique comme les sels de diéthanolamine, de triéthanolamine, de diéthyléthano lamine.

Les polyoléfines à terminaison succinique estérifiée ou amidifiée sont des produits de réaction de (a) une polyoléfine à terminaison succinique, et de (b) une amine ou un alcool, pour former une amide ou un ester.

Le terme « amine » utilisé ici comprend tous types d'amines dont les alcanolamines. Il peut s'agir par exemple de mono-amines primaires, secondaires ou tertiaires, ces amines pouvant être aliphatiques, cycloaliphatiques, aromatiques, hétérocycliques, saturées ou insaturées.

Comme exemple d'alcanolamines, on peut notamment citer la diéthyléthano lamine, et la triéthanolamine.

Par ailleurs, les alcools peuvent être des mono- ou poly-alcools.

Les mono-alcools comprennent les alcools aliphatiques primaires, secondaires ou tertiaires, et les phénols.

Les poly-alcools peuvent être par exemple choisis parmi les poly-alcools aliphatiques, cycloaliphatiques, aromatiques et hétérocycliques.

Comme exemples de polyalcool, on peut notamment citer le glycérol.

Les polyoléfines à terminaison succinique modifiée (estérifiée ou amidifiée) et leur procédé de préparation sont décrits en particulier dans le document US-A-4,708,753. On utilise de préférence des polyoléfines à terminaison succinique estérifiée.

Comme polyoléfines à terminaison succinique, on peut citer notamment les polyisobutylènes à terminaison succinique modifiée, notamment estérifiée, par exemple par la diéthanolamine, et leurs sels, notamment les sels de diéthanolamine, tels que les produits commercialisés sous les dénominations Lubrizol^{®} 2724, Lubrizol^{®} 2722 et Lubrizol^{®} 5603 par la société Lubrizol.

Un autre exemple de polyoléfine à partie polaire utilisable dans l'invention est le produit de la réaction de l'anhydride maléique avec le polyisobutylène, tel que les produits commercialisés sous les dénominations Glissopal^{®} (Glissopal^{®} 2300, 1300 et 1000) (nom INCI : polyisobutene) par la société BASF.

La polyoléfine à partie(s) polaire(s) particulièrement préférée est un produit réactionnel de l'anhydride polyisobuténylsuccinique avec de la diéthyléthanolamine, formant ainsi un sel de diéthyléthanolamine de polybutène succinate de 2-(N, N diéthyl)-amino éthyle.

Ce produit est vendu par exemple sous la dénomination Lubrizol^{®} 5603 par la société Lubrizol, et peut être représenté par la formule suivante : 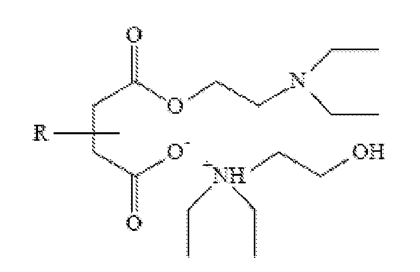 dans laquelle R représente un groupe polyisobutényle, notamment de masse moléculaire en poids de 1000 g/mol. Ce produit a pour nom INCI : hydroxyethyldiethonium polyisobutenyl triethylaminosuccinate (and) diethyl ethanolamine.

Une autre polyoléfine à partie(s) polaire(s) particulièrement envisagée est un ester de polyisobutényl succinate de diéthanolaminoéthyle et de triéthanolamnine. Ce produit est vendu par exemple sous la dénomination Chemccinate^{®} 2000 par la société Chemron.

Comme polyoléfine à partie(s) polaire(s), on peut également utiliser un ester de polyisobutényl succinate de glycéryle, notamment celui vendu sous la dénomination Chemccinate^{®} 1000 AF par la société Chemron.

Les compositions selon l'invention peuvent comprendre de 0,01 % à 10 % en poids, en particulier de 0,1 % à 7 % en poids, et mieux encore de 0,2 % à 5 % en poids de polyoléfine(s) à partie(s) polaire(s) par rapport au poids total de la composition.

Les polyoléfines à partie(s) polaire(s) selon l'invention peuvent être par exemple utilisées comme additif dans une émulsion, et être dans ce cas solubilisées dans la phase huileuse de celle-ci.

Elles peuvent également être utilisées comme émulsionnant et permettre la formation d'émulsions eau-dans-huile (E/H) telles que décrites dans la demande FR 2 811 565.

### Polymères amphiphiles comportant au moins un motif acide acrylamido 2-méthylpropane sulfonique (AMPS)

Les polymères amphiphiles comportant au moins un motif acide acrylamido 2-méthylpropane sulfonique (AMPS) utilisables dans la présente invention, également appelés plus simplement « polymères d'AMPS amphiphiles » dans la suite, comportent à la fois une partie hydrophile et une partie hydrophobe comportant au moins une chaîne grasse.

La chaîne grasse présente dans lesdits polymères d'AMPS amphiphiles selon l'invention peut comporter de préférence de 7 à 30 atomes de carbone, et plus préférentiellement de 7 à 22 atomes de carbone.

Les polymères d'AMPS amphiphiles selon l'invention sont notamment choisis parmi les polymères amphiphiles d'au moins un monomère acide acrylamidon-méthylpropane sulfonique (AMPS) et d'au moins un co-monomère à insaturation éthylénique comportant au moins une partie hydrophobe ayant de 7 à 30 atomes de carbone, en particulier de 7 à 22 atomes de carbone, voire de 12 à 22 atomes de carbone.

Les polymères d'AMPS amphiphiles selon l'invention ont en général un poids moléculaire en poids allant de 50 000 à 10 000 000 g/mol, en particulier de 100 000 à 8 000 000 g/mol et encore plus particulièrement de 100 000 à 7 000 000 g/mol.

Ils peuvent être réticulés ou non réticulés.

Lorsque les polymères d'AMPS amphiphiles selon l'invention sont réticulés, les agents de réticulation peuvent être choisis parmi les composés à polyinsaturation oléfinique couramment utilisés pour la réticulation des polymères obtenus par polymérisation radicalaire.

On peut citer par exemple comme agents de réticulation, le divinylbenzène, l'éther diallylique, le dipropylèneglycol-diallyléther, les polyglycol-diallyléthers, le triéthylèneglycol-divinyléther, l'hydroquinone-diallyl-éther, le di(méth)acrylate d'éthylèneglycol ou de tétraéthylèneglycol, le triméthylol propane triacrylate, le méthylène-bis-acrylamide, le méthylène-bis-méthacrylamide, la triallylamine, le triallylcyanurate, le diallylmaléate, la tétraallyléthylènediamine, le tétra-allyloxy-éthane, le triméthylolpropane-diallyléther, le (méth)acrylate d'allyle, les éthers allyliques d'alcools de la série des sucres, ou d'autres allyl- ou vinyl- éthers d'alcools polyfonctionnels, ainsi que les esters allyliques des dérivés de l'acide phosphorique et/ou vinylphosphonique, ou les mélanges de ces composés.

Les agents de réticulation peuvent être notamment choisis parmi le méthylène-bis-acrylamide, le méthacrylate d'allyle ou le triméthylol propane triacrylate (TMPTA).

Le taux de réticulation peut varier par exemple de 0,01 à 10% en moles et de préférence de 0,2 à 2% en moles par rapport au polymère.

Les polymères d'AMPS amphiphiles selon l'invention peuvent notamment être choisis parmi les polymères amphiphiles statistiques d'AMPS modifiés par réaction avec une n-monoalkylamine ou une di-n-alkylamine en C₆-C₂₂ tels que ceux décrits dans la demande de brevet WO 00/31154.

Un polymère amphiphile convenant à l'invention peut comprendre au moins un monomère hydrophile à insaturation éthylénique choisi par exemple parmi l'acide acrylique, l'acide méthacrylique ou leurs dérivés alkyl substitués ou leurs esters obtenus avec des mono- ou poly-alkylèneglycols, l'acrylamide, le méthacrylamide, la vinylpyrrolidone, le vinylformamide, l'anhydride maléique, l'acide itaconique ou l'acide maléique ou leurs mélanges.

Un polymère amphiphile selon l'invention peut comprendre au moins un co-monomère hydrophobe à insaturation éthylénique.

Un polymère amphiphile convenant à l'invention peut comprendre au moins une partie hydrophobe choisie parmi les radicaux alkyles linéaires, saturés ou insaturés, comme par exemple le n-octyle, le n-décyle, le n-hexadécyle, le n-dodécyle, l'oléyle, ramifiés comme par exemple l'isostéarique ou cycliques comme par exemple le cyclododécane ou l'adamantane.

Un polymère d'AMPS amphiphile peut contenir en outre au moins un co-monomère hydrophobe à insaturation éthylènique, comprenant par exemple :
- un radical fluoré ou alkylfluoré en C₇-C₁₈ (par exemple le groupement de formule -(CH₂)₂-(CF₂)₉-CF₃),
- un radical cholestéryle ou un radical dérivé de cholestérol (par exemple l'hexanoate de cholestéryle),
- un groupe polycyclique aromatique comme le naphtalène ou le pyrène,
- un radical siliconé ou alkylsiliconé ou encore alkylfluorosiliconé.

Ces copolymères sont notamment décrits dans le document EP-A-750899, le brevet US-A-5089578 et dans les publications de Yotaro Morishima suivantes :
- « Self-assembling amphiphilic polyelectrolytes and their nanostructures - Chinese Journal of Polymer Science Vol. 18, N°40, (2000), 323-336 »;
- « Micelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 -3694-3704 » ;
- « Solution properties of micelle networks formed by non-ionic moieties covalently bound to an polyelectrolyte : salt effects on rheological behavior - Langmuir, 2000,Vol.16, N°12, 5324-5332» ;
- « Stimuli responsive amphiphilic copolymers of sodium 2-(acrylamido)-2-methylpropanesulfonate and associative macromonomers - Polym. Preprint, Div. Polym. Chem. 1999, 40(2), 220-221 ».

Ils sont également décrits dans les documents EP 1 069 142, WO 02/44224, WO 02/44225, WO 02/44227, WO 02/44229, WO 02/44230, WO 02/44231, WO 02/44267, WO 02/44268, WO 02/44269, WO 02/44270, WO 02/44271, WO 02/43677, WO 02/43686, WO 02/43687, WO 02/43688, WO 02/43689, au nom de CLARIANT.

Un co-monomère hydrophobe à insaturation éthylénique de l'invention peut être choisi de préférence parmi les acrylates ou les acrylamides de formule (1) suivante : 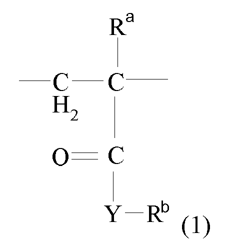 dans laquelle :
- R^{a} désigne un atome d'hydrogène, un radical alkyle linéaire ou ramifié en C₁-C₆, de préférence méthyle ;
- Y désigne O ou NH ;
- R^{b} désigne un radical hydrophobe comportant une chaîne grasse ayant de 7 à 30 atomes de carbone, et de préférence de 7 à 22, et plus particulièrement de 12 à 22 atomes de carbone.

Le radical hydrophobe R^{b} est choisi parmi les radicaux alkyles linéaires en C₇-C₂₂, saturés ou insaturés (par exemple n-octyle, n-décyle, n-hexadécyle, n-dodécyle, oléyle), ramifiés (par exemple isostéarique) ou cycliques (par exemple cyclododécane ou adamantane) ; les radicaux alkylperfluorés en C₇-C₁₈ (par exemple le groupement de formule -(CH₂)₂-(CF₂)₉-CF₃) ; le radical cholestéryle ou un ester de cholestérol comme l'hexanoate de cholestéryle ; les groupes polycycliques aromatiques comme le naphtalène ou le pyrène.

Parmi ces radicaux, les radicaux alkyles linéaires et ramifiés sont plus particulièrement préférés.

Selon un mode de réalisation préféré de l'invention, le radical hydrophobe R^{b} peut comporter en plus au moins un motif oxyde d'alkylène et de préférence une chaîne polyoxyalkylénée.

La chaîne polyoxyalkylénée peut être de façon préférentielle constituée de motifs oxyde d'éthylène et/ou de motifs oxyde de propylène et encore plus particulièrement constituée uniquement de motifs oxyde d'éthylène.

Le nombre de moles de motifs oxyalkylénés peut varier en général de 1 à 30 moles et plus préférentiellement de 1 à 25 moles et encore plus préférentiellement de 3 à 20 moles.

Parmi ces polymères, on peut citer :
- les copolymères réticulés ou non réticulés, neutralisés ou non, comportant de 15 à 60 % en poids de motifs AMPS et de 40 à 85 % en poids de motifs (C₈-₁₆)alkyl(méth)acrylamide ou de motifs (C₈-C₁₆)alkyl(méth)acrylate par rapport au polymère, tels que ceux décrits dans la demande EP-A-750 899 ;
- les terpolymères comportant de 10 à 90% en mole de motifs acrylamide, de 0,1 à 10% en mole de motifs AMPS et de 5 à 80% en mole de motifs n-(C₆-C₁₈)alkylacrylamide, par rapport au polymère, tels que ceux décrits dans le brevet US-A-5,089,578 ;
- les copolymères non réticulés d'AMPS partiellement ou totalement neutralisé et de méthacrylate de n-dodécyle, de n-hexadécyle ou de n-octadécyle, tels que ceux décrits dans les articles de Morishima cités ci-dessus ;
- les copolymères réticulés ou non réticulés d'AMPS partiellement ou totalement neutralisé et de n-dodécylméthacrylamide, tels que ceux décrits dans les articles de Morishima cités ci-dessus.

Comme polymères d'AMPS amphiphiles, on peut également citer les copolymères d'AMPS totalement neutralisé et de méthacrylate de n-dodécyle, de n-hexadécyle et/ou de n-octadécyle, ainsi que les copolymères d'AMPS et de n-dodécylméthacrylamide, non réticulés et réticulés.

On citera plus particulièrement les copolymères d'AMPS amphiphiles réticulés ou non réticulés constitués :
(a) de motifs acide 2-acrylamido 2-méthylpropane sulfonique (AMPS) de formule (2) suivante : 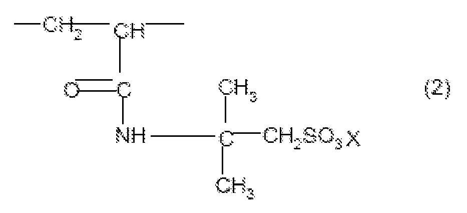 dans laquelle X est un proton, un cation de métal alcalin, un cation alcalino-terreux ou l'ion ammonium ;
(b) et de motifs de formule (3) suivante : 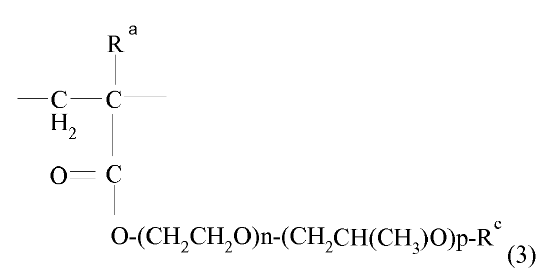
dans laquelle n et p, indépendamment l'un de l'autre désignent un nombre de moles et varient de 0 à 30, de préférence de 1 à 25 et plus préférentiellement de 3 à 20 sous réserve que n + p soit inférieur ou égal à 30, de préférence inférieur à 25 et encore mieux inférieur à 20 ; R^{a} désigne un atome d'hydrogène, un radical alkyle linéaire ou ramifié en C₁-C₆, de préférence méthyle et R^{c} désigne un alkyle linéaire ou ramifié comportant de 7 à 22 atomes de carbone, de préférence de 12 à 22 atomes de carbone.

Dans la formule (2), le cation X désigne plus particulièrement le sodium ou l'ammonium.

Parmi les monomères de formule (3) on peut citer :
- les esters d'acide (méth)acrylique et d'alcool gras en C₁₀-C₁₈ polyoxethylénés à 8 OE comme le produit GENAPOL C-080^{®} vendu par la Société CLARIANT,
- les esters d'acide (méth)acrylique et d'oxoalcool gras en C₁₁ polyoxyéthyléné à 8 OE comme le produit GENAPOL UD-080^{®} vendu par la Société CLARIANT,
- les esters d'acide (méth)acrylique et d'alcool gras polyoxyéthyléné en C₁₂-C₁₄ à 7 OE comme le produit GENAPOL LA-070^{®} vendu par la Société CLARIANT,
- les esters d'acide (méth)acrylique et d'alcool gras polyoxyéthyléné en C₁₂-C₁₄ à 11 OE comme le produit GENAPOL LA-110^{®} vendu par la Société CLARIANT,
- les esters d'acide (méth)acrylique et d'alcool gras polyoxyéthyléné en C₁₆-C₁₈ à 8 OE comme le produit GENAPOL T-080^{®} vendu par la Société CLARIANT,
- les esters d'acide (méth)acrylique et d'alcool gras polyoxyéthyléné en C₁₆-C₁₈ à 15 OE comme le produit GENAPOL T-150^{®} vendu par la Société CLARIANT,
- les esters d'acide (méth)acrylique et d'alcool gras polyoxyéthyléné en C₁₆-C₁₈ à 11 OE comme le produit GENAPOL T-110^{®} vendu par la Société CLARIANT,
- les esters d'acide (méth)acrylique et d'alcool gras polyoxyéthyléné en C₁₆-C₁₈ à 20 OE comme le produit GENAPOL T-200^{®} vendu par la Société CLARIANT,
- les esters d'acide (méth)acrylique et d'alcool gras polyoxyéthyléné en C₁₆-C₁₈ à 25 OE comme le produit GENAPOL T-250^{®} vendu par la Société CLARIANT,
- les esters d'acide (méth)acrylique et d'alcool gras polyoxyéthyléné en C₁₈-C₂₂ à 25 OE et/ ou d'isoalcool gras polyoxyéthyléné en C₁₆-C₁₈ à 25 OE .

On choisira plus particulièrement :
i. ceux non réticulés pour lesquels p= 0, n = 7 ou 25, R^{a} désigne un méthyle et R^{c} représente un mélange d'alkyle en C₁₂-C₁₄ ou en C₁₆-C₁₈,
ii. ceux réticulés pour lesquels p = 0, n = 8 ou 25, R^{a} désigne un méthyle et R^{c} représente un mélange d'alkyle en C₁₆-C₁₈.

Ces polymères sont décrits et synthétisés dans la demande EP1069142.

Ces polymères d'AMPS amphiphiles particuliers peuvent être obtenus selon les procédés classiques de polymérisation radicalaire en présence d'un ou plusieurs initiateurs tels que par exemple, l'azobisisobutyronitrile (AIBN), l'azobisdiméthylvaléronitrile, le chlorhydrate de 2,2-azobis-[2-amidinopropane] (ABAH - 2,2-Azo-Bis-[2-Amidinopropane] Hydrochloride), les peroxydes organiques tels que le peroxyde de dilauryle, le peroxyde de benzoyle, l'hydropéroxyde de tert-butyle, des composés peroxydés minéraux tels que le persulfate de potassium ou d'ammonium, ou H₂O₂ éventuellement en présence de réducteurs.

Ces polymères d'AMPS amphiphiles peuvent être notamment obtenus par polymérisation radicalaire en milieu tert-butanol dans lequel ils précipitent. En utilisant la polymérisation par précipitation dans le tert-butanol, il est possible d'obtenir une distribution de la taille des particules du polymère particulièrement favorable pour ses utilisations.

La réaction peut être conduite à une température comprise entre 0 et 150°C, de préférence entre 10 et 100°C, soit à pression atmosphérique, soit sous pression réduite.

Elle peut aussi être réalisée sous atmosphère inerte, et de préférence sous azote.

Les polymères d'AMPS amphiphiles selon l'invention peuvent de préférence être neutralisés partiellement ou totalement par une base minérale telle la soude, la potasse, l'ammoniaque ou une base organique telle que la mono-, di- ou tri-éthanolamine, un aminométhylpropanediol, la N-méthyl-glucamine, les acides aminés basiques comme l'arginine et la lysine, et les mélanges de ces composés. Ils peuvent notamment être totalement ou pratiquement totalement neutralisés, c'est-à-dire neutralisés à au moins 80 %.

La concentration molaire en % des motifs de formule (2) et des motifs de formule (3) dans les polymères d'AMPS amphiphiles selon l'invention peut varier en fonction de l'application cosmétique souhaitée, par exemple de la nature de l'émulsion (huile-dans-eau ou eau-dans-huile) et des propriétés rhéologiques de la formulation recherchées. Elle peut par exemple varier entre 0,1 et 99,9 % en moles.

Les polymères d'AMPS amphiphiles selon l'invention peu hydrophobes seront plus appropriés pour l'épaississement et/ou la stabilisation des émulsions huile-dans-eau.

La proportion molaire en motifs de formule (3) peut alors varier de préférence de 0,1 à 50 % en moles, plus particulièrement de 1 à 25 % en moles et encore plus particulièrement de 3 à 10% en moles.

Les polymères d'AMPS amphiphiles selon l'invention plus hydrophobes seront plus appropriés pour l'épaississement et/ou la stabilisation des émulsions eau-dans-huile.

La proportion molaire en motifs de formule (3) peut alors varier de préférence de 50,1 à 99,9 % en moles, plus particulièrement de 60 à 95 % en moles et encore plus particulièrement de 65 à 90 % en moles.

La distribution des monomères dans les polymères d'AMPS amphiphiles selon l'invention peut être, par exemple, alternée, bloc (y compris multibloc) ou quelconque.

A titre indicatif et sans que cela soit limitatif, on peut citer notamment le copolymère d'AMPS et de méthacrylate d'alcool en C₁₂-C₁₄ éthoxylé (copolymère non réticulé obtenu à partir de Genapol LA-070 et d'AMPS) (nom CTFA : Ammonium Acryloyldiméthyltaurate/Laureth-7 méthacrylate copolymer) commercialisé sous la dénomination ARTISTOFLEX LNC par la société Clariant, le copolymère d'AMPS et de méthacrylate de stéaryle éthoxylé (25 EO) (copolymère réticulé par le triméthylolpropane triacrylate, obtenu à partir de Genapol T-250 et d'AMPS) (nom CTFA : Ammonium Acryloyldimethyltaurate / Steareth-25 Methacrylate Crosspolymer) commercialisé sous la dénomination ARISTOFLEX HMS par la société Clariant, l'Aristoflex SNC (copolymère AMPS/méthacrylate d'alcool C₁₆/C₁₈ éthoxylés (8 moles EP 80/20 ; nom CTFA : Ammonium acryloyldiméthyltaurate/stéareth-8 mzthacrylate copolymer) et l'Aristoflex HMB (copolymère AMPS/méthacrylate de béhényl éthoxyle (25 OE), réticulé par le triméthylolpropane triacrylate (TMPTA).

Les polymères d'AMPS amphiphiles selon l'invention peuvent être présents en des quantités en matière active allant de 0,01 à 20 % en poids, plus préférentiellement de 0,1 à 10 % en poids, encore plus préférentiellement de 0,1 à 5 % en poids et plus particulièrement encore de 0,3 à 2 % en poids par rapport au poids total de la composition.

Les polymères d'AMPS amphiphiles selon l'invention peuvent être utilisés comme additif dans une émulsion, et être dans ce cas solubilisés préférentiellement dans la phase aqueuse de celle-ci.

Elle peuvent également être utilisées comme émulsionnant, et permettre la formation d'émulsion huile-dans-eau (H/E).

### DERIVES C-GLYCOSIDES

Un dérivé C-glycoside convenant à l'invention peut être un composé de formule générale (I) suivante : 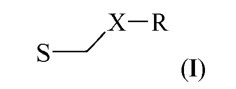 dans laquelle :
- R représente :
   - un radical alkyle linéaire, saturé en C₁ à C₂₀, en particulier en C₁ à C₁₀, ou insaturé en C₂ à C₂₀, en particulier en C₂ à C₁₀, ou un radical alkyle ramifié ou cyclique, saturé ou insaturé, en C₃ à C₂₀, en particulier en C₃ à C₁₀;
   - un radical hydrofluoro- ou perfluoro-alkyle, linéaire saturé en C₁ à C₂₀, en particulier en C₁ à C₁₀, ou insaturé en C₂ à C₂₀, en particulier en C₂ à C₁₀, ou ramifié ou cyclique, saturé ou insaturé, en C₃ à C₂₀, en particulier en C₃ à C₁₀;
      la chaîne hydrocarbonée constituant lesdits radicaux pouvant, le cas échéant, être interrompue par 1, 2, 3 ou plus d'hétéroatomes choisis parmi :

   - un oxygène,
   - un soufre,
   - un azote, et
   - un silicium,
      et pouvant être éventuellement substituée par au moins un radical choisi parmi :
   - -OR₄,
   - -SR₄,
   - -NR₄R₅,
   - -COOR₄,
   - -CONHR₄,
   - -CN,
   - un atome d'halogène,
   - un radical hydrofluoro- ou perfluoro-alkyle, en C₁ à C₆, et/ou
   - un radical cycloalkyle en C₃ à C₈,
   avec R₄ et R₅ pouvant représenter, indépendamment l'un de l'autre, un atome d'hydrogène, ou un radical alkyle, perfluoroalkyle ou hydrofluoroalkyle linéaire, saturé en C₁ à C₃₀, notamment en C₁ à C₁₂, ou insaturé en C₂ à C₃₀, notamment en C₂ à C₁₂, ou ramifié ou cyclique, saturé ou insaturé, en C₃ à C₃₀, notamment en C₃ à C₁₂ ; ou un radical aryle en C₆ à C₁₀,
- X représente un radical choisi parmi les groupements : 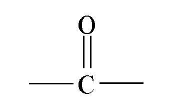 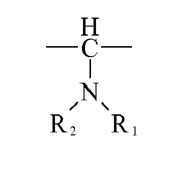 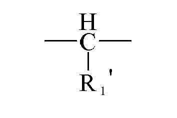 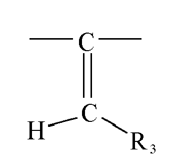 avec R₁, R₂ et R₃ représentant, indépendamment l'un de l'autre, un atome d'hydrogène, ou un radical R, avec R tel que défini précédemment, et R'₁ représente un atome d'hydrogène, un groupe -OH ou un radical R tel que défini précédemment, R₁ pouvant désigner également un radical aryle en C₆ à C₁₀ ;
- S représente un monosaccharide ou un polysaccharide comportant jusqu'à 20 unités sucre, en particulier jusqu'à 6 unités sucre, sous forme pyranose et/ou furanose et de série L et/ou D, ledit mono- ou poly-saccharide pouvant être substitué par un groupement hydroxyle obligatoirement libre, et éventuellement une ou plusieurs fonction(s) amine(s) éventuellement protégée(s), et
- la liaison S-CH₂-X représente une liaison de nature C-anomérique, qui peut être α ou β,
   ainsi que leurs sels cosmétiquement acceptables, leurs solvates tels que les hydrates et leurs isomères.

Dans le cadre de la présente invention, par « halogène », on entend le chlore, le fluor, le brome ou l'iode.

Le terme « aryle » désigne un cycle aromatique tel que phényle, éventuellement substitué par un ou plusieurs radicaux alkyles en C₁-C₄.

Le terme « cycloalkyle en C₃ à C₈ » désigne un cycle aliphatique ayant de 3 à 8 atomes de carbone, incluant par exemple le cyclopropyle, le cyclopentyle et le cyclohéxyle.

Parmi les groupes alkyle convenant à la mise en oeuvre de l'invention, on peut notamment citer les groupes méthyle, éthyle, isopropyle, n-propyle, n-butyle, t-butyle, isobutyle, sec-butyle, pentyle, n-hexyle, cyclopropyle, cyclopentyle, cyclohexyle, et allyle.

Selon un mode de réalisation de l'invention, on peut utiliser un dérivé C-glycoside répondant à la formule (I) pour lequel S peut représenter un monosaccharide ou un polysaccharide contenant jusqu'à 6 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions amine obligatoirement protégée, X et R conservant par ailleurs l'ensemble des définitions précédemment données.

Avantageusement, un monosaccharide de l'invention peut être choisi parmi le D-glucose, le D-galactose, le D-mannose, le D-xylose, le D-lyxose, le L-fucose, le L-arabinose, le L-rhamnose, l'acide D-glucuronique, l'acide D-galacturonique, l'acide D-iduronique, la N-acétyl-D-glucosamine, la N-acétyl-D-galactosamine et désigne avantageusement le D-glucose, le D-xylose, la N-acétyl-D-glucosamine ou le L-fucose, et en particulier le D-xylose.

Plus particulièrement, un polysaccharide de l'invention contenant jusqu'à 6 unités sucre peut être choisi parmi le D-maltose, le D-lactose, le D-cellobiose, le D-maltotriose, un disaccharide associant un acide uronique choisi parmi l'acide D-iduronique ou l'acide D-glucuronique avec une hexosamine choisie parmi la D-galactosamine, la D-glucosamine, la N-acétyl-D-galactosamine, la N-acétyl-D-glucosamine, un oligosaccharide contenant au moins un xylose qui peut être avantageusement choisi parmi le xylobiose, le méthyl-β-xylobioside, le xylotriose, le xylotétraose, le xylopentaose et le xylohexaose et notamment le xylobiose qui est composé de deux molécules de xylose liées par une liaison 1-4.

Plus particulièrement, S peut représenter un monosaccharide choisi parmi le D-glucose, le D-xylose, le L-fucose, le D-galactose, le D-maltose et notamment le D-xylose.

Selon un autre mode de réalisation de l'invention, on peut utiliser des dérivés C-glycoside répondant à la formule (I) pour lesquels X représente un groupement choisi parmi -CO-, -CH(OH)-, -CH(NR₁R₂)-, -CH(R)-, en particulier -CO-, -CH(OH)-, -CH(NH₂)-, -CH(NHCH₂CH₂CH₂OH)-, -CH(NHPh)-, -CH(CH₃)-, et plus particulièrement un groupement -CO-, -CH(OH)-, -CH(NH₂)-, et préférentiellement un groupement - CH(OH)-, S et R conservant par ailleurs l'ensemble des définitions précédemment données.

Selon un autre mode de réalisation de l'invention, on peut utiliser un dérivé C-glycoside répondant à la formule (I) pour lesquels R représente un radical alkyle linéaire, saturé en C₁ à C₂₀, en particulier en C₁ à C₁₀, ou insaturé en C₂ à C₂₀, en particulier en C₂ à C₁₀, ou un radical alkyle ramifié ou cyclique, saturé ou insaturé, en C₃ à C₂₀ ; en particulier en C₃ à C₁₀, et éventuellement substitué comme décrit précédemment, S et X conservant par ailleurs l'ensemble des définitions précédemment données. De préférence, R désigne un radical linéaire en C₁-C₄, notamment C₁-C₃, éventuellement substitué par - OH, -COOH ou -COOR"₂, R"₂ étant un radical alkyle saturé en C₁-C₄, notamment éthyle. Préférentiellement R désigne un radical alkyle linéaire non substitué en C₁-C₄, notamment C₁-C₂, en particulier éthyle.

Parmi les dérivés C-glycosides de formule (I), on utilise de préférence ceux pour lesquels :
- R représente un radical alkyle en linéaire, saturé en C₁ à C₂₀, en particulier en C₁ à C₁₀, ou insaturé en C₂ à C₂₀, en particulier en C₂ à C₁₀, ou un radical alkyle ramifié ou cyclique, saturé ou insaturé, en C₃ à C₂₀ ; en particulier en C₃ à C₁₀, et éventuellement substitué comme décrit précédemment ;
- S représente un monosaccharide comme décrit précédemment ;
- X représente -CO-, -CH(OH)-, -CH(NR₁R₂)-, -CH(R)- comme décrit précédemment.

De préférence, on utilise un dérivé C-glycoside de formule (I) pour lesquels :
- R désigne un radical linéaire en C₁-C₄, notamment C₁-C₃, éventuellement subsitué par -OH, -COOH ou -COOR"₂, R"₂ étant un radical alkyle saturé en C₁-C₄, notamment éthyle ;
- S représente un monosaccharide comme décrit précédemment ;
- X représente un groupement choisi parmi -CO-, -CH(OH)-, -C(NH₂)-, - CH(NHCH₂CH₂CH₂OH)-, -CH(NHPh)-, -CH(CH₃)-, et plus particulièrement un groupement -CO-, -CH(OH)-, -CH(NH₂)-, et préférentiellement un groupement -CH(OH)-.

Préférentiellement, on utilise un dérivé C-glycoside de formule (I) pour lesquels :
- R désigne un radical alkyle linéaire non substitué en C₁-C₄, notamment C₁-C₂, en particulier éthyle ;
- S représente un monosaccharide comme décrit précédemment ; notamment le D-glucose, le D-xylose, la N-acétyl-D-glucosamine ou le L-fucose, et en particulier le D-xylose ;
- X représente un groupement choisi parmi -CO-, -CH(OH)-, -CH(NH₂)-, et préférentiellement un groupement -CH(OH)-.

Les sels acceptables pour l'usage non thérapeutique des composés décrits dans la présente invention comprennent des sels non toxiques conventionnels desdits composés tels que ceux formés à partir d'acides organiques ou inorganiques. A titre d'exemple, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Lorsque le composé de formule (I) comporte un groupe acide, la neutralisation du ou des groupes acides peut être effectuée par une base minérale, telle que LiOH, NaOH, KOH, Ca(OH)₂, NH₄OH, Mg(OH)₂ ou Zn(OH)₂; ou par une base organique telle qu'une alkylamine primaire, secondaire ou tertiaire, par exemple la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine, la diméthylamino-2-propanol, le 2-amino-2-(hydroxyméthyl)-1,3-propanediol. On peut encore citer la lysine ou la 3-(diméthylamino)propylamine.

Les solvates acceptables pour les composés décrits dans la présente invention comprennent des solvates conventionnels tels que ceux formés lors de la dernière étape de préparation desdits composés du fait de la présence de solvants. A titre d'exemple, on peut citer les solvates dus à la présence d'eau ou d'alcools linéaires ou ramifiés comme l'éthanol ou l'isopropanol.

Parmi les dérivés C-glycosides de formule (I), utilisés selon l'invention, on considère tout particulièrement :
1. C-β-D-xylopyranoside-n-propane-2-one ;
2. C-α-D-xylopyranoside-n-propane-2-one ;
3. 1-[2-(3-hydroxy-propylamino)-propyl]-C-β-D-xylopyranose ;
4. 1-[2-(3-hydroxy-propylamino)-propyl]-C-α-D-xylopyranose ;
5. C-β-D-xylopyranoside-2-hydroxy-propane ;
6. C-α-D-xylopyranoside-2-hydroxy-propane ;
7. C-β-D-xylopyranoside-2-amino-propane ;
8. C-α-D-xylopyranoside-2-amino-propane ;
9. C-β-D-xylopyranoside-2-phénylamino-propane ;
10. C-α-D-xylopyranoside-2-phénylamino-propane ;
11. ester éthylique de l'acide 3-méthyl-4-(C-β-D-xylopyranoside)-butyrique ;
12. ester éthylique de l'acide 3-méthyl-4-(C-α-D-xylopyranoside)-butyrique ;
13. acide 6-(C-β-D-xylopyranoside)-5-céto-hexanoique ;
14. acide 6-(C-α-D-xylopyranoside)-5-céto-hexanoique ;
15. acide 6-(C-β-D-xylopyranoside)-5-hydroxy-hexanoique ;
16. acide 6-(C-α-D-xylopyranoside)-5-hydroxy-hexanoique ;
17. acide 6-(C-β-D-xylopyranoside)-5-amino-hexanoique ;
18. acide 6-(C-α-D-xylopyranoside)-5-amino-hexanoique ;
19. acide 6-(C-β-D-xylopyranoside)-5-phenylamino-hexanoique ;
20. acide 6-(C-α-D-xylopyranoside)-5-phenylamino-hexanoique ;
21. 1-(C-β-D-xylopyranoside)-hexane-2,6-diol ;
22. 1-(C-α-D-xylopyranoside)-hexane-2,6-diol ;
23. acide 5-(C-β-D-xylopyranoside)-4-céto-pentanoique ;
24. acide 5-(C-α-D-xylopyranoside)-4-céto-pentanoique ;
25. acide 5-(C-β-D-xylopyranoside)-4-hydroxy-pentanoique ;
26. acide 5-(C-α-D-xylopyranoside)-4-hydroxy-pentanoique ;
27. acide 5-(C-β-D-xylopyranoside)-4-amino-pentanoique ;
28. acide 5-(C-α-D-xylopyranoside)-4-amino-pentanoique ;
29. acide 5-(C-β-D-xylopyranoside)-4-phénylamino-pentanoique ;
30. acide 5-(C-α-D-xylopyranoside)-4-phénylamino-pentanoique ;
31. 1-(C-β-D-xylopyranoside)-pentane-2,5-diol ;
32. 1-(C-α-D-xylopyranoside)-pentane-2,5-diol ;
33. 1-(C-β-D-fucopyranoside)-propane-2-one ;
34. 1-(C-α-D-fucopyranoside)-propane-2-one ;
35. 1-(C-β-L-fucopyranoside)-propane-2-one ;
36. 1-(C-α-L-fucopyranoside)-propane-2-one ;
37. 1-(C-β-D-fucopyranoside)-2-hydroxy-propane ;
38. 1-(C-α-D-fucopyranoside)-2-hydroxy-propane ;
39. 1-(C-β-L-fucopyranoside)-2-hydroxy-propane ;
40. 1-(C-α-L-fucopyranoside)-2-hydroxy-propane ;
41. 1-(C-β-D-fucopyranoside)-2-amino-propane ;
42. 1-(C-α-D-fucopyranoside)-2-amino-propane ;
43. 1-(C-β-L-fucopyranoside)-2-amino-propane ;
44. 1-(C-α-L-fucopyranoside)-2-amino-propane ;
45. 1-(C-β-D-fucopyranoside)-2-phénylamino-propane ;
46. 1-(C-α-D-fucopyranoside)-2-phénylamino-propane ;
47. 1-(C-β-L-fucopyranoside)-2-phénylamino-propane ;
48. 1-(C-α-L-fucopyranoside)-2-phénylamino-propane ;
49. ester éthylique de l'acide 3-méthyl-4-(C-β-D-fucopyranoside)-butyrique ;
50. ester éthylique de l'acide 3-méthyl-4-(C-α-D-fucopyranoside)-butyrique ;
51. ester éthylique de l'acide 3-méthyl-4-(C-β-L-fucopyranoside)-butyrique ;
52. ester éthylique de l'acide 3-méthyl-4-(C-α-L-fucopyranoside)-butyrique ;
53. acide 6-(C-β-D-fucopyranoside)-5-céto-hexanoique ;
54. acide 6-(C-α-D-fucopyranoside)-5-céto-hexanoique ;
55. acide 6-(C-β-L-fucopyranoside)-5-céto-hexanoique ;
56. acide 6-(C-α-L-fucopyranoside)-5-céto-hexanoique ;
57. acide 6-(C-β-D-fucopyranoside)-5-hydroxy-hexanoique ;
58. acide 6-(C-α-D-fucopyranoside)-5-hydroxy-hexanoique ;
59. acide 6-(C-β-L-fucopyranoside)-5-hydroxy-hexanoique ;
60. acide 6-(C-α-L-fucopyranoside)-5-hydroxy-hexanoique ;
61. acide 6-(C-β-D-fucopyranoside)-5-amino-hexanoique ;
62. acide 6-(C-α-D-fucopyranoside)-5-amino-hexanoique ;
63. acide 6-(C-β-L-fucopyranoside)-5-amino-hexanoique ;
64. acide 6-(C-α-L-fucopyranoside)-5-amino-hexanoique ;
65. 1-(C-β-D-fucopyranoside)-hexane-2,6-diol ;
66. 1-(C-α-D-fucopyranoside)-hexane-2,6-diol ;
67. 1-(C-β-L-fucopyranoside)-hexane-2,6-diol ;
68. 1-(C-α-L-fucopyranoside)-hexane-2,6-diol ;
69. acide 5-(C-β-D-fucopyranoside)-4-céto-pentanoique ;
70. acide 5-(C-α-D-fucopyranoside)-4-céto-pentanoique ;
71. acide 5-(C-β-L-fucopyranoside)- 4-céto-pentanoique ;
72. acide 5-(C-α-L-fucopyranoside)- 4-céto-pentanoique ;
73. acide 5-(C-β-D-fucopyranoside)-4-hydroxy-pentanoique ;
74. acide 5-(C-α-D-fucopyranoside)-4-hydroxy-pentanoique ;
75. acide 5-(C-β-L-fucopyranoside)-4-hydroxy-pentanoique ;
76. acide 5-(C-α-L-fucopyranoside)-4-hydroxy-pentanoique ;
77. acide 5-(C-β-D-fucopyranoside)-4-amino-pentanoique ;
78. acide 5-(C-α-D-fucopyranoside)-4-amino-pentanoique
79. acide 5-(C-β-L-fucopyranoside)-4-amino-pentanoique ;
80. acide 5-(C-α-L-fucopyranoside)-4-amino-pentanoique ;
81. 1-(C-β-D-fucopyranoside)-pentane-2,5-diol ;
82. 1-(C-α-D-fucopyranoside)-pentane-2,5-diol ;
83. 1-(C-β-L-fucopyranoside)-pentane-2,5-diol,
84. 1-(C-α-L-fucopyranoside)-pentane-2,5-diol ;
85. 1-(C-β-D-glucopyranosyl)-2-hydroxy-propane ;
86. 1-(C-α-D-glucopyranosyl)-2-hydroxy-propane ;
87. 1-(C-β-D-glucopyranosyl)-2-amino-propane ;
88. 1-(C-α-D-glucopyranosyl)-2-amino-propane ;
89. 1-(C-β-D-glucopyranosyl)-2-phénylamino-propane ;
90. 1-(C-α-D-glucopyranosyl)-2-phénylamino-propane ;
91. ester éthylique de l'acide 3-méthyl-4-(C-β-D-glucopyranosyl)-butyrique ;
92. ester éthylique de l'acide 3-méthyl-4-(C-α-D-glucopyranosyl)-butyrique ;
93. acide 6-(C-β-D-glucopyranosyl)-5-céto-hexanoique ;
94. acide 6-(C-α-D-glucopyranosyl)-5-céto-hexanoique ;
95. acide 6-(C-β-D-glucopyranosyl)-5-hydroxy-hexanoique ;
96. acide 6-(C-α-D-glucopyranosyl)-5-hydroxy-hexanoique ;
97. acide 6-(C-β-D-glucopyranosyl)-5-amino-hexanoique ;
98. acide 6-(C-α-D-glucopyranosyl)-5-amino-hexanoique ;
99. acide 6-(C-β-D-glucopyranosyl)-5-phénylamino-hexanoique ;
100. acide 6-(C-α-D-glucopyranosyl)-5-phénylamino-hexanoique ;
101. 1-(C-β-D-glucopyranosyl)-hexane-2,6-diol ;
102. 1-(C-α-D-glucopyranosyl)-hexane-2,6-diol ;
103. acide 6-(C-β-D-glucopyranosyl)-5-céto-pentanoique ;
104. acide 6-(C-α-D-glucopyranosyl)-5-céto-pentanoique ;
105. acide 6-(C-β-D-glucopyranosyl)-5-hydroxy-pentanoique ;
106. acide 6-(C-α-D-glucopyranosyl)-5-hydroxy-pentanoique ;
107. acide 6-(C-β-D-glucopyranosyl)-5-amino-pentanoique ;
108. acide 6-(C-α-D-glucopyranosyl)-5-hydroxy-pentanoique ;
109. acide 6-(C-β-D-glucopyranosyl)-5-phénylamino-pentanoique ;
110. acide 6-(C-α-D-glucopyranosyl)-5-phénylamino-pentanoique ;
111. 1-(C-β-D-glucopyranosyl)-pentane-2,5-diol;
112. 1-(C-α-D-glucopyranosyl)-pentane-2,5-diol,
113. 1-(C-β-D-galactopyranosyl)-2-hydroxy-propane ;
114. 1-(C-α-D-galactopyranosyl)-2-hydroxy-propane ;
115. 1-(C-β-D-galactopyranosyl)-2-amino-propane ;
116. 1-(C-α-D-galactopyranosyl)-2-amino-propane ;
117. 1-(C-β-D-galactopyranosyl)-2-phénylamino-propane ;
118. 1-(C-α-D-galactopyranosyl)-2-phénylamino-propane ;
119. ester éthylique de l'acide 3-méthyl-4-(β-D-galactopyranosyl)-butyrique ;
120. ester éthylique de l'acide 3-méthyl-4-(α-D-galactopyranosyl)-butyrique ;
121. acide 6-(C-β-D-galactopyranosyl)-5-céto-hexanoique ;
122. acide 6-(C-α-D-galactopyranosyl)-5-céto-hexanoique ;
123. acide 6-(C-β-D-galactopyranosyl)-5-hydroxy-hexanoique ;
124. acide 6-(C-α-D-galactopyranosyl)-5-hydroxy-hexanoique ;
125. acide 6-(C-β-D-galactopyranosyl)-5-amino-hexanoique ;
126. acide 6-(C-α-D-galactopyranosyl)-5-amino-hexanoique ;
127. acide 6-(C-β-D-galactopyranosyl)5-phénylamino-hexanoique ;
128. acide 6-(C-α-D-galactopyranosyl)5-phénylamino-hexanoique ;
129. 1-(C-β-D-galactopyranosyl)-hexane-2,6-diol,
130. 1-(C-α-D-galactopyranosyl)-hexane-2,6-diol ;
131. acide 6-(C-β-D-galactopyranosyl)-5-céto-pentanoique ;
132. acide 6-(C-α-D-galactopyranosyl)-5-céto-pentanoique ;
133. acide 6-(C-β-D-galactopyranosyl)-5-hydroxy-pentanoique ;
134. acide 6-(C-α-D-galactopyranosyl)-5-hydroxy-pentanoique ;
135. acide 6-(C-β-D-galactopyranosyl)-5-amino-pentanoique ;
136. acide 6-(C-α-D-galactopyranosyl)-5-amino-pentanoique ;
137. acide 6-(C-β-D-galactopyranosyl)-5-phénylamino-pentanoique ;
138. acide 6-(C-α-D-galactopyranosyl)-5-phénylamino-pentanoique ;
139. 1-(C-β-D-galactopyranosyl)-pentane-2,6-diol ;
140. 1-(C-α-D-galactopyranosyl)-pentane-2,6-diol ;
141. 1-(C-β-D-fucofuranosyl)-propane-2-one ;
142. 1-(C-α-D-fucofuranosyl)-propane-2-one ;
143. 1-(C-β-L-fucofuranosyl)-propane-2-one ;
144. 1-(C-α-L-fucofuranosyl)-propane-2-one ;
145. 3'-(acétamido-C-β-D-glucopyranosyl)-propane-2'-one ;
146. 3'-(acétamido-C-α-D-glucopyranosyl)-propane-2'-one ;
147. 1-(acétamido-C-β-D-glucopyranosyl)-2-hydroxyl-propane ;
148. 1-(acétamido-C-β-D-glucopyranosyl)-2-amino-propane ;
149. 1-(acétamido-C-β-D-glucopyranosyl)-2-phénylamino-propane ;
150. 1-(acétamido-C-α-D-glucopyranosyl)-2-phénylamino-propane ;
151. ester éthylique de l'acide 3-méthyl-4-(acétamido-C-β-D-glucopyranosyl)-butyrique ;
152. ester éthylique de l'acide 3-méthyl-4-(acétamido-C-α-D-glucopyranosyl)-butyrique ;
153. acide 6-(acétamido-C-β-D-glucopyranosyl)-5-céto-hexanoique ;
154. acide 6-(acétamido-C-α-D-glucopyranosyl)-5-céto-hexanoique ;
155. acide 6-(acétamido-C-β-D-glucopyranosyl)-5-hydroxy-hexanoique ;
156. acide 6-(acétamido-C-α-D-glucopyranosyl)-5-hydroxy-hexanoique ;
157. acide 6-(acétamido-C-β-D-glucopyranosyl)-5-amino-hexanoique ;
158. acide 6-(acétamido-C-α-D-glucopyranosyl)-5-amino-hexanoique ;
159. acide 6-(acétamido-C-β-D-glucopyranosyl)5-phénylamino-hexanoique ;
160. acide 6-(acétamido-C-α-D-glucopyranosyl)5-phénylamino-hexanoique ;
161. 1-(acétamido-C-β-D-glucopyranosyl)-hexane-2,6-diol ;
162. 1-(acétamido-C-α-D-glucopyranosyl)-hexane-2,6-diol ;
163. acide 6-(acétamido-C-β-D-glucopyranosyl)-5-céto-pentanoique ;
164. acide 6-(acétamido-C-α-D-glucopyranosyl)-5-céto-pentanoique ;
165. acide 6-(acétamido-C-β-D-glucopyranosyl)-5-hydroxy- pentanoique ;
166. acide 6-(acétamido-C-α-D-glucopyranosyl)-5-hydroxy- pentanoique ;
167. acide 6-(acétamido-C-β-D-glucopyranosyl)-5-amino- pentanoique ;
168. acide 6-(acétamido-C-α-D-glucopyranosyl)-5-amino- pentanoique ;
169. acide 6-(acétamido-C-β-D-glucopyranosyl)-5-phénylamino- pentanoique ;
170. acide 6-(acétamido-C-α-D-glucopyranosyl)-5-phénylamino- pentanoique ;
171. 1-(acétamido-C-β-D-glucopyranosyl)-pentane-2,5-diol;
172. 1-(acétamido-C-α-D-glucopyranosyl)-pentane-2,5-diol.

A titre illustratif et non limitatif des dérivés C-glycoside convenant plus particulièrement à l'invention, on peut notamment citer les dérivés suivants :
- le C-β-D-xylopyranoside-n-propane-2-one,
- le C-α- D-xylopyranoside-n-propane-2-one,
- le C-β-D-xylopyranoside-2-hydroxy-propane,
- le C-α- D-xylopyranoside-2-hydroxy-propane,
- la 1-(C-β-D-fucopyranoside)-propane-2-one,
- la 1-(C-α-D-fucopyranoside)-propane-2-one,
- la 1-(C-β-L-fucopyranoside)-propane-2-one,
- la 1-(C-α-L-fucopyranoside)-propane-2-one,
- le 1 -(C-β-D-fucopyranoside)-2-hydroxy-propane,
- le 1-(C-α-D-fucopyranoside)-2-hydroxy-propane,
- le 1-(C-β-L-fucopyranoside)-2-hydroxy-propane,
- le 1-(C-α-L-fucopyranoside) -2-hydroxy-propane,
- le 1-(C-β-D-Glucopyranosyl)-2-hydroxyl-propane,
- le 1-(C-α-D-Glucopyranosyl)-2-hydroxyl-propane,
- le 1-(C-β-D-galactopyranosyl)-2-hydroxyl-propane,
- le 1-(C-α-D-galactopyranosyl)-2-hydroxyl-propane
- la 1-(C-β-D-fucofuranosyl)-propane-2-one,
- la 1-(C-α-D-fucofuranosyl)-propane-2-one
- la 1-(C-β-L-fucofuranosyl)-propane-2-one,
- la 1-(C-α-L-fucofuranosyl)-propane-2-one,
- le C-β-D-maltopyranoside-n-propane-2-one,
- le C-α-D-maltopyranoside-n-propane-2-one
- le C-β-D-maltopyranoside-2-hydroxy-propane,
- le C-α-D-maltopyranoside-2-hydroxy-propane, leurs isomères et leurs mélanges.

Selon un mode de réalisation, le C-β-D-xylopyranoside-2-hydroxy-propane ou le C-α-D-xylopyranoside-2-hydroxy-propane, et mieux le C-β-D-xylopyranoside-2-hydroxy-propane, peuvent être avantageusement mis en oeuvre pour la préparation d'une composition selon l'invention.

Selon un mode de réalisation particulier, le dérivé C-glycoside est le C-β-D-xylopyranoside-2-hydroxy-propane sous forme de solution à 30 % en matière active dans un mélange eau/propylèneglycol (60/40 % en poids) tel que le produit fabriqué par CHIMEX sous la dénomination commerciale « MEXORYL SBB^{®} ».

Bien entendu, selon l'invention, un dérivé C-glycoside répondant à la formule (I) peut être utilisé seul ou en mélange avec d'autres dérivés C-glycosides et en toutes proportions.

Un dérivé de C-glycoside convenant à l'invention peut notamment être obtenu par la méthode de synthèse décrite dans le document WO 02/051828.

La quantité de dérivé C-glycoside à mettre en oeuvre dans une composition selon l'invention dépend de l'effet cosmétique ou thérapeutique recherché, et peut donc varier dans une large mesure.

L'homme de l'art peut aisément, sur la base de ses connaissances générales, déterminer les quantités appropriées.

Une composition selon l'invention peut comprendre environ 0,0001 % à environ 25 % en poids de matière active par rapport au poids total de la composition, et en particulier d'environ 0,001 % à environ 10 % en poids de matière active et plus particulièrement d'environ 0,05 % à environ 5 % en poids de dérivé C-glycoside par rapport au poids total de la composition.

Une composition conforme à l'invention comprend un milieu physiologiquement acceptable.

Ce milieu physiologiquement acceptable peut au moins comprendre une phase aqueuse en mélange ou non avec un ou plusieurs solvants organiques tels qu'un alcool en C₁-C₈, notamment l'éthanol, l'isopropanol, le tert-butanol, le n-butanol, des polyols comme la glycérine, le propylène glycol, le butylène glycol, et des éthers de polyol.

Une composition selon l'invention peut être anhydre.

On entend par « composition anhydre » toute composition comprenant moins de 5 % d'eau par rapport au poids total de la composition et plus préférentiellement moins de 1 % d'eau.

Une composition selon l'invention peut également comprendre une phase grasse, qui peut comprendre des huiles, des gommes, des cires usuellement utilisées dans le domaine d'application considéré.

Ainsi selon un mode de réalisation une composition selon l'invention peut en outre comprendre au moins une phase grasse choisie parmi une phase grasse solide à température ambiante (20-25 °C) et pression atmosphérique, une phase grasse liquide, et un mélange de celles-ci.

Une phase grasse liquide convenant à la mise en oeuvre de l'invention peut comprendre une huile volatile, une huile non volatile, et un mélange de celles-ci. Une huile volatile ou non volatile peut être une huile hydrocarbonée, notamment d'origine animale ou végétale, une huile synthétique, une huile siliconée, une huile fluorée ou un mélange de celles-ci.

Une phase grasse solide convenant à la mise en oeuvre de l'invention peut être, par exemple, choisie parmi les corps gras pâteux, les gommes, et leurs mélanges.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Lorsqu'une composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants autres que les polymères émulsionnants utilisés dans la présente demande, qui peuvent être utilisés dans la composition sous forme d'émulsion sont choisis parmi ceux classiquement utilisés dans le domaine cosmétique.

Outre les polymères émulsionnants selon l'invention, les compositions selon l'invention peuvent comprendre de 0,3 % à 30 % en poids, et en particulier de 0,5 à 20 % en poids d'émulsionnant(s) et/ou de coémulsionnant(s) par rapport au poids total de la composition.

Une émulsion selon l'invention peut, en outre, contenir des vésicules lipidiques.

Lorsqu'une composition selon l'invention est une solution ou un gel huileux, la phase grasse peut représenter plus de 90 % du poids total de la composition.

Une composition selon l'invention peut contenir également des adjuvants habituels dans le domaine considéré, tels que d'autres tensioactifs ou émulsionnants, des gélifiants hydrophiles ou lipophiles, des additifs hydrophiles ou lipophiles, des conservateurs, des antioxydants, des solvants, des parfums, des charges, des filtres UVA et ou UVB (organique ou inorganique), des pigments, des fibres, des agents chélateurs, des absorbeurs d'odeur, des matières colorantes, et d'autres actifs cosmétiques ou pharmaceutiques.

Les quantités de ces différents adjuvants sont celles classiquement utilisées dans le domaine cosmétique, et peuvent être par exemple de 0,01 % à 10 % du poids total de la composition. Ces adjuvants, selon leur nature, peuvent être introduits dans la phase grasse, dans la phase aqueuse et/ou dans les sphérules lipidiques.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, polyéthylène.

Une composition de l'invention peut se présenter sous toutes les formes galéniques envisageables.

Notamment, une composition selon l'invention peut avoir la forme d'une solution aqueuse, alcoolique, hydroalcoolique; d'une dispersion du type lotion ou sérum; d'une émulsion eau-dans-huile, huile-dans-eau ou multiple; d'une suspension; de microcapsules ou microparticules; de dispersions vésiculaires de type ionique et/ou non ionique; d'une lotion aqueuse, huileuse ou sous forme de sérum; de capsules, de granulés, de sirops, de comprimés; de mousse, de préparation solide; de composition pour aérosol comprenant également un agent propulseur sous pression.

Une composition selon l'invention peut se présenter sous la forme d'une composition pour soin capillaire, notamment un shampooing, une lotion de mise en plis, une lotion traitante, une crème ou un gel coiffant, une composition de teinture, notamment d'oxydation, des lotions restructurantes pour les cheveux, une composition de permanente (notamment une composition pour le premier temps d'une permanente), une lotion ou un gel antichute, un shampooing antiparasitaire.

Elle peut également se présenter sous la forme d'une composition de nettoyage, de protection, de traitement ou de soin pour le visage, pour les mains, pour les pieds, pour les grands plis anatomiques ou pour le corps (par exemple crèmes de jour, crème de nuit, crème démaquillante, composition anti-solaire, lait corporels de protection ou de soin, laits après-solaire, lotion, gel ou mousse pour le soin de la peau, comme des lotion de nettoyage, composition de bronzage artificiel); une composition de maquillage du corps ou du visage telle qu'un fond de teint; une composition pour le bain; une composition désodorisante comprenant par exemple un agent bactéricide; une composition après-rasage; une composition épilatoire; une composition contre les piqûres d'insectes; une composition anti-douleur; une composition pour traiter certaines maladies de la peau comme l'eczéma, la rosacée, le psoriasis, les lichens, les prurits sévères.

Lorsqu'une composition selon l'invention est destinée à un usage de type peeling, elle peut également se présenter sous toutes les formes galéniques évoquées ci-dessus pour autant qu'elle s'élimine facilement par rinçage, et notamment sous forme de gel aqueux, de solution aqueuse ou hydroalcoolique.

Une composition selon l'invention peut être appliquée par tout moyen permettant une répartition uniforme et notamment à l'aide d'un coton, d'une tige, d'un pinceau, d'une gaze, d'une spatule ou d'un tampon, ou encore par pulvérisation, et peut être éliminée par rinçage à l'eau ou à l'aide d'un détergent doux.

Une composition selon l'invention peut en outre comprendre un ou des actif(s) cosmétique(s) ou thérapeutique(s) additionnel(s), comme par exemple des agents anti-âge/anti-rides (tels que les agents anti-glycation, stimulant la synthèse de macromolécules dermiques ou épidermiques et/ou empêchant leur dégradation, stimulants la prolifération des fibroblastes et/ou des kératinocytes ou stimulant la différenciation des kératinocytes, les myorelaxants), des agents hydratants, des agents desquamants, des agents anti-pollution et anti-radicalaires, des agents amincissants, des agents agissant sur la microcirculation, les agents agissant sur le métabolisme énergétiques des cellules, les tenseurs, les agents dépigmentants ou pro-pigmentants, les agents desquamants, des agents anti-acné ou encore, des agents anti-inflammatoires/anti-irritants.

On peut encore citer tous les actifs connus pour leur activité sur le vieillissement de la peau comme les agents kératolytiques ou prodésquamants, par exemple les α-hydroxy-acides, les β-hydroxy-acides, les α-céto-acides, les rétinoïdes et leurs esters, le rétinol, l'acide rétinoïque et ses dérivés ; les vitamines C, B3 ou PP, B5, E et les dérivés de ces vitamines et notamment leurs esters, la vitamine K et ses dérivés (K1, K2,...) ; les agents anti-radicaux libres; la DHEA et ses dérivés; le coenzyme Q10; les agents blanchissants et dépigmentants comme l'acide kojique, les dérivés de para-aminophénols, l'arbutine et leurs dérivés, et leurs mélanges.

Une composition selon l'invention peut être destinée à une application cosmétique de soin et/ou de maquillage et/ou dermatologique.

Les exemples ci-après sont donnés à titre d'illustration de l'invention et ne doivent pas être interprétés comme limitant sa portée.

### EXEMPLES

Le dérivé C-glycoside utilisé est le C-β-D-xylopyranoside-2-hydroxy-propane commercialisé sous la dénomination MEXORYL SBB^{®} de CHIMEX. Il se présente sous la forme d'une solution à 30 % en poids en matière active (MA) dans un mélange eau/1,2-propanediol 60/40.

### Exemple 1 :

Solution aqueuse selon l'invention comprenant un dérivé C-glycoside et, à titre de polymère émulsionnant, un polymère d'AMPS amphiphile selon l'invention.

| | |
|---|---|
| Copolymère AMPS/méthacrylate d'alcool C₁₂/C₂₄ ethoxylé (8 moles EO) 80/20 (Aristoflex LNC^{®} de chez CLARIANT) | 1 % |
| Eau distillée | qsp 100 % |
| C-β-D-xylopyranoside-2-hydroxy-propane | 2 % (MA) |

| Exemple comparatif: | |
|---|---|
| Eau distillée | qsp 100 % |
| C-β-D-xylopyranoside-2-hydroxy-propane | 2 % (MA) |

La méthode d'analyse utilisée est celle décrite dans l'exemple 10 de la demande WO 02/051828 (modèle tridimensionnel de peau artificielle).

La néosynthèse de glycosaminoglycanes est significativement plus importante en utilisant la composition de l'exemple 1 qu'avec celle décrite dans l'exemple comparatif.

### Exemple 2 :

Emulsion huile-dans-eau comprenant un dérivé C-glycoside et, à titre de polymère émulsionnant, un polymère d'AMPS amphiphile selon l'invention.

| Phase 1 | |
|---|---|
| Huile d'amande d'abricot | 7 % |
| Isononanoate d'isononyle | 7 % |
| Cyclométhicone | 7 % |
| Parfum | 0,5 % |

| Phase 2 | |
|---|---|
| Eau distillée | qsp 100 % |
| C-β-D-xylopyranoside-2-hydroxy-propane | 5 % (MA) |
| Copolymère AMPS/méthacrylate d'alcool C12/C24 ethoxylé (8 moles EO) 80/20 (Aristoflex LNC^{®} de chez CLARIANT) | 1 % |
| Isostéarate de glycérol | 1 % |
| Conservateurs | 0,5 % |

| | |
|---|---|
| MA : matière active. | |

La phase 1 est ajoutée à la phase 2 dans laquelle le produit Aristoflex LNC a été préalablement hydraté, à température ambiante, et sous agitation vive à l'aide d'un rotor-stator, pendant 30 min.

La composition ainsi obtenue est sous la forme d'une émulsion eau-dans-huile, très fine et stable.

### Exemple 3 :

Emulsion eau-dans-huile comprenant un dérivé C-Glycoside et, à titre de polymère émulsionnant, par une polyoléfine à partie(s) polaire(s).

| *Phase 1 :* | |
|---|---|
| Isohexadécane | 7 % |
| Cyclopentasiloxane | 5 % |
| Isododécane | 2 % |
| Mélange de polyisobutényl succinate de triéthanolamine-diéthanolamine et de palmitate de2-éthylhexyle (vendu sous la référence Chemccinate 2000^{®} par la société CHEMRON) | 3,5 % |
| Conservateur | 0,3% |

| *Phase 2 :* | |
|---|---|
| C-β-D-xylopyranoside-2-hydroxy-propane | 5 % MA |
| Sulfate de magnésium | 0,8 % |
| Conservateurs | 0,5% |
| Eau | qsp 100 % |

| | |
|---|---|
| MA : matière active. | |

La phase 2 est introduite dans la phase 1sous agitation vive.

La composition ainsi obtenue est sous la forme d'une émulsion eau-dans-huile fluide.

## Revendications

1. Composition cosmétique et/ou dermatologique comprenant, dans un milieu physiologiquement acceptable, au moins un dérivé C-glycoside et au moins un polymère émulsionnant choisi parmi les polyoléfines à partie(s) polaire(s) et les polymères amphiphiles comportant au moins un motif acide acrylamido-2-méthyl propane sulfonique (AMPS).

2. Composition selon la revendication précédente, dans laquelle le dérivé C-glycoside répond à la formule générale (I) suivante : 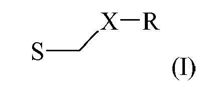 dans laquelle :
- R représente :
- un radical alkyle linéaire, saturé en C₁ à C₂₀, en particulier en C₁ à C₁₀, insaturé en C₂ à C₂₀, en particulier en C₂ à C₁₀, ou un radical alkyle ramifié ou cyclique, saturé ou insaturé, en C₃ à C₂₀, en particulier en C₃ à C₁₀ ;
- un radical hydrofluoro- ou perfluoro-alkyle, linéaire saturé en C₁ à C₂₀, en particulier en C₁ à C₁₀, ou insaturé en C₂ à C₂₀, en particulier en C₂ à C₁₀, ou ramifié ou cyclique, saturé ou insaturé, en C₃ à C₂₀, en particulier en C₃ à C₁₀ ;
la chaîne hydrocarbonée constituant lesdits radicaux pouvant, le cas échéant, être interrompue par 1, 2, 3 ou plus d'hétéroatomes choisis parmi :
- un oxygène,
- un soufre,
- un azote, et
- un silicium,
et pouvant être éventuellement substituée par au moins un radical choisi parmi :
- -OR₄,
- -SR₄,
- -NR₄R₅,
- -COOR₄,
- -CONHR₄,
- -CN,
- un atome d'halogène,
- un radical hydrofluoro- ou perfluoro-alkyle, en C₁ à C₆, et/ou
- un radical cycloalkyle en C₃ à C₈,
avec R₄ et R₅ pouvant représenter, indépendamment l'un de l'autre, un atome d'hydrogène, ou un radical alkyle, perfluoroalkyle ou hydrofluoroalkyle linéaire, saturé en C₁ à C₃₀, notamment en C₁ à C₁₂, ou insaturé en C₂ à C₃₀, notamment en C₂ à C₁₂, ou ramifié ou cyclique, saturé ou insaturé, en C₃ à C₃₀, notamment en C₃ à C₁₂ ; ou un radical aryle en C₆ à C₁₀,
- X représente un radical choisi parmi les groupements : 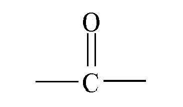 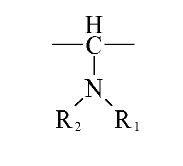 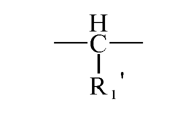 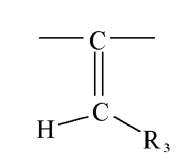 avec R₁, R₂ et R₃ représentant, indépendamment l'un de l'autre, un atome d'hydrogène, ou un radical R, avec R tel que défini précédemment, et R'₁ représentant un atome d'hydrogène, un groupe -OH ou un radical R tel que défini précédemment, R₁ pouvant désigner également un radical aryle en C₆ à C₁₀,
- S représente un monosaccharide ou un polysaccharide comportant jusqu'à 20 unités sucre, en particulier jusqu'à 6 unités sucre, sous forme pyranose et/ou furanose et de série L et/ou D, ledit mono- ou poly-saccharide pouvant être substitué par un groupement hydroxyle obligatoirement libre, et éventuellement une ou plusieurs fonction(s) amine(s) éventuellement protégée(s), et
- la liaison S-CH₂-X représente une liaison de nature C-anomérique, qui peut être α ou β,
ainsi que leurs sels cosmétiquement acceptables, leurs solvates tels que les hydrates et leurs isomères.

3. Composition selon la revendication précédente, dans laquelle S représente un monosaccharide choisi parmi le D-glucose, le D-xylose, le L-fucose, le D-galactose, le D-maltose et notamment le D-xylose.

4. Composition selon la revendication 2 ou 3, dans laquelle X représente un groupement choisi parmi -CO-, -CH(OH)-, -CH(NH₂)-, et préférentiellement un groupement -CH(OH)-.

5. Composition selon l'une quelconque des revendications 2 à 4, dans laquelle R désigne un radical linéaire en C₁-C₄, notamment C₁-C₃, éventuellement substitué par -OH, -COOH ou -COOR"₂, R"₂ étant un radical alkyle saturé en C₁-C₄, notamment éthyle.

6. Composition selon l'une quelconque des revendications précédentes, dans laquelle le dérivé C-glycoside est choisi parmi :
- le C-β-D-xylopyranoside-n-propane-2-one,
- le C-α- D-xylopyranoside-n-propane-2-one,
- le C-β-D-xylopyranoside-2-hydroxy-propane,
- le C-α- D-xylopyranoside-2-hydroxy-propane,
- la 1-(C-β-D-fucopyranoside)-propane-2-one,
- la 1-(C-α-D-fucopyranoside)-propane-2-one,
- la 1-(C-β-L-fucopyranoside)-propane-2-one,
- la 1-(C-α-L-fucopyranoside)-propane-2-one,
- le 1-(C-β-D-fucopyranoside)-2-hydroxy-propane,
- le 1-(C-α-D-fucopyranoside)-2-hydroxy-propane,
- le 1-(C-β-L-fucopyranoside)-2-hydroxy-propane,
- le 1-(C-α-L-fucopyranoside) -2-hydroxy-propane,
- le 1-(C-β-D-Glucopyranosyl)-2-hydroxyl-propane,
- le 1-(C-α-D-Glucopyranosyl)-2-hydroxyl-propane,
- le 1-(C-β-D-galactopyranosyl)-2-hydroxyl-propane,
- le 1-(C-α-D-galactopyranosyl)-2-hydroxyl-propane
- la 1-(C-β-D-fucofuranosyl)-propane-2-one,
- la 1-(C-α-D-fucofuranosyl)-propane-2-one
- la 1-(C-β-L-fucofuranosyl)-propane-2-one,
- la 1-(C-α-L-fucofuranosyl)-propane-2-one,
- le C-β-D-maltopyranoside-n-propane-2-one,
- le C-α-D-maltopyranoside-n-propane-2-one
- le C-β-D-maltopyranoside-2-hydroxy-propane,
- le C-α-D-maltopyranoside-2-hydroxy-propane, leurs isomères et leurs mélanges.

7. Composition selon l'une quelconque des revendications précédentes dans laquelle le dérivé C-glycoside est choisi parmi le C-β-D-xylopyranoside-2-hydroxy-propane et le C-α-D-xylopyranoside-2-hydroxy-propane, et est plus particulièrement le C-β-D-xylopyranoside-2-hydroxy-propane.

8. Composition selon l'une quelconque des revendications précédentes, comprenant le dérivé C-glycoside à raison d'environ 0,00001 % à environ 25 % en poids de matière active, en particulier d'environ 0,001 % à environ 10 % en poids et plus particulièrement d'environ 0,05 % à environ 5 % en poids, par rapport au poids total de la composition.

9. Composition selon l'une quelconque des revendications précédentes, dans laquelle le polymère émulsionnant est une polyoléfine à partie(s) polaire(s).

10. Composition selon la revendication précédente, dans laquelle la polyoléfine à partie(s) polaire(s) comporte une partie apolaire polyoléfinique comprenant au moins 40 atomes de carbone, et en particulier de 60 à 700 atomes de carbone.

11. Composition selon la revendication précédente, dans laquelle la partie apolaire polyoléfinique est choisie parmi les oligomères, les polymères et/ou les copolymères de monomères en C₂ à C₂₀ et notamment d'éthylène, de propylène, de 1-butène, d'isobutène, de 1-pentène, de 2-méthyl-1-butène, de 3-méthyl-1-butène, de 1-héxène, de 1-heptène, de 1-octène, de 1-décène, de 1-undécène, de 1-dodécène, de 1-tridécène, de 1-tétradécène, de 1-pentadécène, de 1-héxadécène, de 1-heptadécène et de 1-octadécène.

12. Composition selon l'une quelconque des revendications 9 à 11, dans laquelle la partie polaire de la polyoléfine est anionique, cationique, non ionique, zwitterionique ou amphotère.

13. Composition selon l'une quelconque des revendications 9 à 11, dans laquelle la partie polaire de la polyoléfine est constituée de polyalkylène-glycols, de polyalkylène-imines, d'acides ou de diacides carboxyliques, de leurs anhydrides ou de leurs dérivés tels que leurs esters, leurs amides, et leurs sels.

14. Composition selon l'une quelconque des revendications 9 à 13, dans laquelle la partie polaire de la polyoléfine est choisie dans le groupe comprenant le polyoxyéthylène, l'acide ou l'anhydride succinique, les esters ou amides de l'acide ou de l'anhydride succinique, les sels de métal alcalin ou alcalino-terreux ou sels organiques de l'acide ou de l'anhydride succinique, ou les sels partiels des monoesters ou monoamides de l'acide ou de l'anhydride succinique.

15. Composition selon l'une quelconque des revendications 9 à 14, dans laquelle la polyoléfine à partie polaire est un polyisobutène à terminaison succinique éventuellement modifiée.

16. Composition selon l'une quelconque des revendications 9 à 15, dans laquelle la polyoléfine à partie polaire est le polyisobutène à terminaison succinique estérifiée.

17. Composition selon l'une quelconque des revendications 9 à 16, comprenant de 0,01 % à 10 % en poids, en particulier de 0,1 % à 7 % en poids, et mieux encore de 0,2 % à 5 % en poids de polyoléfine(s) à partie(s) polaire(s) par rapport au poids total de la composition.

18. Composition selon l'une quelconque des revendications 1 à 8, dans laquelle le polymère émulsionnant comprend au moins un polymère amphiphile comportant au moins un motif acide acrylamido 2-méthyl propane sulfonique (AMPS).

19. Composition selon la revendication précédente, dans laquelle le polymère émulsionnant est un polymère amphiphile d'au moins un monomère acide acrylamido 2-méthylpropane sulfonique (AMPS) et d'au moins un co-monomère à insaturation éthylénique comportant au moins une partie hydrophobe ayant de 7 à 30 atomes de carbone, en particulier de 7 à 22 atomes de carbone, voire de 12 à 22 atomes de carbone.

20. Composition selon la revendication précédente, dans laquelle le polymère amphiphile comprend au moins un co-monomère hydrophile à insaturation éthylénique choisi parmi l'acide acrylique, l'acide méthacrylique ou leurs dérivés alkylsubstitués ou leurs esters obtenus avec des mono- ou poly-alkylèneglycols, l'acrylamide, le méthacrylamide, la vinylpyrrolidone, le vinylformamide, l'anhydride maléique, l'acide itaconique ou l'acide maléique.

21. Composition selon la revendication 19 ou 20, dans laquelle la partie hydrophobe est choisie parmi les radicaux alkyles linéaires, saturés ou insaturés, comme par exemple le n-octyle, le n-décyle, le n-héxadécyle, le n-dodécyle, l'oléyle, ramifiés, comme par exemple isostéarique et cycliques comme par exemple le cyclododécane ou l'adamantane.

22. Composition selon l'une quelconque des revendications 19 à 21, dans laquelle ledit co-monomère hydrophobe à insaturation éthylénique est choisi parmi les acrylates ou les acrylamides de formule (1) suivante : 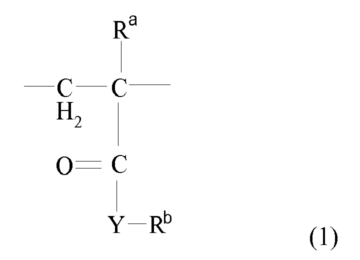 dans laquelle :
- R^{a} désigne un atome d'hydrogène, un radical alkyle linéaire ou ramifié en C₁-C₆, de préférence méthyle ;
- Y désigne O ou NH ;
- R^{b} désigne un radical hydrophobe comportant une chaîne grasse ayant de 7 à 30 atomes de carbone, et de préférence de 7 à 22, et plus particulièrement de 12 à 22 atomes de carbone.

23. Composition selon la revendication précédente, dans laquelle R^{b} est choisi parmi les radicaux alkyles linéaires en C₇-C₂₂, saturés ou insaturés, par exemple n-octyle, n-décyle, n-hexadécyle, n-dodécyle, oléyle, ramifiés, par exemple isostéarique, ou cycliques, par exemple cyclododécane ou adamantane; les radicaux alkylperfluorés en C₇-C₁₈ (par exemple le groupement de formule -(CH₂)₂-(CF₂)₉-CF₃ ; le radical cholestéryle ou un ester de cholestérol comme l'hexanoate de cholestéryle ; les groupes polycycliques aromatiques comme le naphtalène ou le pyrène.

24. Composition selon l'une quelconque des revendications 18 à 23, dans laquelle le polymère émulsionnant est choisi parmi les copolymères d'AMPS amphiphiles réticulés ou non réticulés constitués :
(a) de motifs acide 2-acrylamido 2-méthyl propane sulfonique de formule (2) suivante : 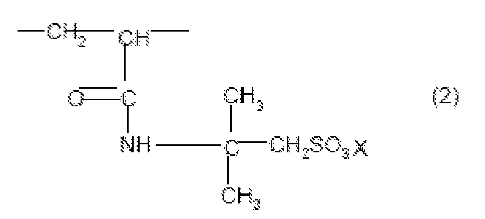 dans laquelle X est un proton, un cation de métal alcalin, un cation alcalino-terreux ou l'ion ammonium, et de préférence le sodium ou l'ammonium, et
(b) de motifs de formule (3) suivante : 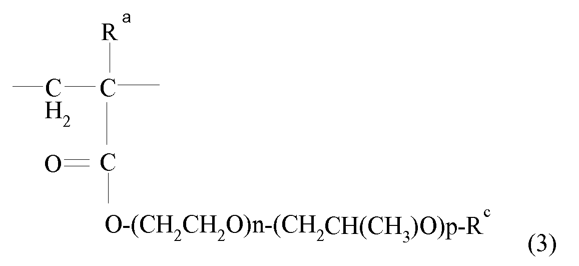
dans laquelle n et p, indépendamment l'un de l'autre désignent un nombre de moles et varient de 0 à 30, de préférence de 1 à 25 et plus préférentiellement de 3 à 20 sous réserve que n + p soit inférieur ou égal à 30, de préférence inférieur à 25 et encore mieux inférieur à 20, R^{a} désigne un atome d'hydrogène, un radical alkyle linéaire ou ramifié en C₁-C₆, de préférence méthyle, et R^{c} désigne un alkyle linéaire ou ramifié comportant de 7 à 22 atomes de carbone, de préférence de 12 à 22 atomes de carbone.

25. Composition selon la revendication précédente, dans laquelle le polymère émulsionnant est non réticulé, et est choisi parmi les copolymères pour lesquels p = 0, n = 7 ou 25, R^{a} désigne un méthyle et R^{c} représente un mélange d'alkyle en C₁₂-C₁₄ ou en C₁₆-C₁₈.

26. Composition selon la revendication 24, dans laquelle le polymère émulsionnant est réticulé, et est choisi parmi les copolymères pour lesquels p = 0, n = 8 ou 25, R^{a} désigne un méthyle et R^{c} représente un mélange d'alkyle en C₁₆-C₁₈.

27. Utilisation d'une association d'au moins un dérivé C-glycoside et d'au moins un polymère émulsionnant choisi parmi les polyoléfines à partie(s) polaire(s) et les polymères amphiphiles comportant au moins un motif acide acrylamido-2-méthyl propane sulfonique (AMPS) à titre d'actif cosmétique destiné à prévenir et/ou lutter contre les signes du vieillissement cutané.

28. Procédé de traitement non thérapeutique de maquillage et/ou de soin des matières kératiniques, notamment de la peau, comprenant au moins l'étape d'appliquer sur lesdites matières kératiniques au moins une couche d'une composition telle que définie selon l'une quelconque des revendications 1 à 26.
